# EUROPEAN PATENT APPLICATION

(11) **EP 3 170 898 A1**
(43) Date of publication of application: **24.05.2017**
(21) Application number: 15822247.1
(22) Date of filing: 14.07.2015
(51) Int. Cl.: C12N 15/09, C12M 1/00, C12N 15/113, C12Q 1/68

(54) **METHOD FOR DETECTING NUCLEIC ACID, CAPTURE PROBE, DETECTION PROBE SET, MICROARRAY, NUCLEIC ACID DETECTION KIT, NUCLEIC-ACID-IMMOBILIZED SOLID PHASE, AND FLUID DEVICE**

(30) Priority: 18.07.2014 JP 2014147726
(71) Applicant: The University of Tokyo, Tokyo 113-8654 (JP); Nikon Corporation, Tokyo 108-6290 (JP)
(72) Inventor: ICHIKI, Takanori, Tokyo 113-8654 (JP); UENO, Taro, Tokyo 113-8654 (JP); FUNATSU, Takashi, Tokyo 113-8654 (JP); IIZUKA, Ryo, Tokyo 113-8654 (JP); SUZUKI, Kuno, Tokyo 108-6290 (JP)
(74) Representative: Hoffmann Eitle
(86) International application number: PCT/JP2015/070192
(87) International publication number: WO 2016/010047

(57) **Abstract**

A method for detecting a nucleic acid includes (a) bringing a solution including a nucleic acid and a detection probe labeled with a labeling substance into contact with a solid phase on which a capture probe is immobilized, the capture probe having a first portion including a sequence capable of hybridizing with the nucleic acid, a second portion including a sequence capable of hybridizing with the detection probe, and a stem portion for forming a double strand; (b) hybridizing the nucleic acid with the first portion, hybridizing the detection probe with the second portion, and forming a nucleic acid-detection probe-capture probe complex on the solid phase; (c) ligating the nucleic acid and the detection probe; (d) eliminating binding between the capture probe and the detection probe that has not been ligated with the nucleic acid; and (e) detecting the labeling substance in the complex.

## Description

### Technical Field

The present invention relates to a method for detecting a nucleic acid, a capture probe, a detection probe set, a microarray, a nucleic acid detection kit, a nucleic-acid-immobilized solid phase, and a fluid device.

Priority is claimed on Japanese Patent Application No. 2014-147726, filed on July 18, 2014, the content of which is incorporated herein by reference.

### Background Art

In the related art, a DNA microarray targeting an mRNA is widely known as means for measuring a gene expression level in cells. In the 21^{st} century, it has been reported that a miRNA, which is a short and non-coding RNA, regulates gene expression in a living body, and a relationship between aberrant expression of the miRNA and a variety of diseases including cancer has been clarified. A development of the DNA microarray targeting a miRNA has been competitively expanded based on the above knowledge.

Furthermore, as it is found that the miRNA circulates in blood, it is suggested that cancer can be diagnosed only by a blood examination and it is estimated that a market of the DNA microarray targeting a miRNA will be expanded in the near future.

Meanwhile, a quantitative PCR (qRT-PCR) method, which uses a reverse transcription reaction to a cDNA, has been widely utilized as a technology for quantifying the miRNA. The quantitative PCR method is a method in which the miRNA is converted into the cDNA using a reverse transcriptase and the cDNA is amplified, so as to estimate the amount of miRNA used as a template. Since the quantitative PCR method is a method for measuring the cDNA amount amplified to a constant amount using a PCR reaction, the quantitative PCR method has high quantitativity compared to the DNA microarray, but has a problem in that a parallel treatment of a plurality of samples or a variety of miRNAs is difficult.

The well-known DNA microarray targeting a miRNA (hereinafter referred as miRNA targeting DNA microarray) is an array in which a nucleic acid probe having a gene sequence which complementarily hybridizes with the target miRNA is arrayed on a substrate.

The following method is exemplified as a method for quantifying a miRNA using the miRNA targeting DNA microarray. First, the miRNA is extracted from a biological sample and the miRNA is fluorescence-labeled, and then added to the miRNA targeting DNA microarray so as to be hybridized with the nucleic acid probe on the substrate. Next, after the miRNA non-specifically adsorbed to the substrate is cleansed, the miRNA amount is estimated using the fluorescence intensity as an index.

When preparing miRNA from a biological sample, the total RNA is extracted from a biological sample and refined, then the total RNA including a miRNA is fluorescence-labeled, and then the fluorescence-labeled total RNA including the fluorescence-labeled miRNA is brought into contact with the miRNA targeting DNA microarray.

However, since the miRNA amount in a living body, in particular, in blood is extremely small at 0.01% by mass in the total RNA, in a case where such a pretreatment is complicated, the miRNA is likely to be adsorbed or decomposed during pipetting. Furthermore, there is also a problem in that a variation in the fluorescence labeling rate for every measurement lowers the reproducibility of the measurement result.

In addition, since such a pretreatment by a manual operation is affected by the technical level of a researcher or clinical laboratory technologist, it is desired that all pretreatment steps be totally automated so as to be performed on a chip using µ-TAS (Micro-Total Analysis Systems) in the future. Here, the µ-TAS means a micro fluid device provided with a tiny flow channel, a reaction chamber, and a mixing chamber on the chip and for analyzing a nucleic acid on one chip using a technology of MEMS (Micro Electro Mechanical Systems).

However, the fluorescence labeling method has been a bottleneck as described below and integration of the pretreatment steps for total automation has not been advanced.

As a major fluorescence labeling method of a miRNA, a method for fluorescence-labeling a base portion of the miRNA directly, or a method for adding a fluorescence-labeled nucleotide to the 3' end of the miRNA using an enzyme such as T4 DNA ligase is exemplified.

However, since all nucleic acids are non-specifically fluorescence-labeled in these methods, it is necessary to eliminate an unreacted fluorescence reagent from the target miRNA which has been fluorescence-labeled in advance, before hybridizing the miRNA with a nucleic acid probe on a substrate. It is general to use gel filtration chromatography for the separation of the target miRNA and the fluorescence reagent, and it is necessary to separate the short miRNA with about 22 bases accurately from the unreacted fluorescence reagent. For example, in a case of the separation using the µ-TAS, a step of moving a biological sample for a long distance to a region clogged with a resin is needed, and it is difficult to conduct this step within a chip whose space is limited. In addition, even if it is possible, in order to repeat the test continuously, a long period of time may be needed to wash out the unreacted fluorescence reagent.

With respect to the aforementioned problem, a sandwich type microarray method has been suggested, which can detect the miRNA without conducing the separation step of the unreacted fluorescent pigment (refer to PTL 1).

### Citation List

### Patent Literature

[PTL 1] PCT International Publication No. WO 2008/052774

### Summary of Invention

### Technical Problem

However, for example, in the method disclosed in PTL 1, since a signal is detected only in a case where the miRNA and 4 types of the probes are totally collided and 5 types of molecules form a complex, the method has a problem in that quantitativity is deteriorated, and the operation is complicated and takes a time.

### Solution to Problem

One embodiment of the present invention suggests the following (1) to (8).
(1) A method for detecting a nucleic acid according to one embodiment of the present invention includes the following steps:
   (a) a step of bringing a solution including a nucleic acid and a detection probe labeled with a labeling substance into contact with a solid phase on which a capture probe is immobilized, the capture probe having a first portion including a sequence capable of hybridizing with the nucleic acid, the second portion including a sequence capable of hybridizing with the detection probe, and a stem portion for forming a double strand;
   (b) a step of hybridizing the nucleic acid with the first portion, hybridizing the detection probe with the second portion, and forming a nucleic acid-detection probe-capture probe complex on the solid phase;
   (c) a step of ligating the nucleic acid and the detection probe in a state where the nucleic acid is hybridized with the first portion and the detection probe is hybridized with the second portion;
   (d) a step of eliminating binding between the capture probe and the detection probe not ligated with the nucleic acid; and
   (e) a step of detecting the labeling substance in the nucleic acid-detection probe-capture probe complex in which the nucleic acid and the detection probe are ligated and which is formed on the solid phase.
(2) A method for detecting a nucleic acid according to one embodiment of the present invention includes the following steps:
   (a') a step of bringing a solution including a nucleic acid into contact with a solid phase on which a capture probe is immobilized, the capture probe having a first portion including a sequence capable of hybridizing with the nucleic acid, a second portion including a sequence capable of hybridizing with a detection probe, and a stem portion for forming a double strand,
      wherein the detection probe labeled with a labeling substance is hybridized with the second portion in advance;
   (b') a step of hybridizing the nucleic acid with the first portion and forming a nucleic acid-detection probe-capture probe complex on the solid phase;
   (c) a step of ligating the nucleic acid and the detection probe in a state where the nucleic acid is hybridized with the first portion and the detection probe is hybridized with the second portion;
   (d) a step of eliminating binding between the capture probe and the detection probe not ligated with the nucleic acid; and
   (e) a step of detecting the labeling substance in the nucleic acid-detection probe-capture probe complex in which the nucleic acid and the detection probe are ligated and which is formed on the solid phase.
(3) A capture probe according to one embodiment of the present invention includes:
   a first portion including a sequence capable of hybridizing with a nucleic acid as a detection target; a second portion including a sequence capable of hybridizing with a detection probe labeled with a labeling substance; and a stem portion for forming a double strand.
(4) A detection probe set according to one embodiment of the present invention includes:
   a plurality of types of detection probes, in which the detection probe can be bound to a second portion of a capture probe, the capture probe includes a first portion including a sequence capable of hybridizing with a nucleic acid as a detection target, the second portion including a sequence capable of hybridizing with the detection probe, and a stem portion for forming a double strand, and the detection probe includes a type of a detection probe which includes a first detection probe and a second detection probe which is labeled by a labeling substance that is different from each other or in a different amount.
(5) A microarray according to one embodiment of the present invention which is formed by a plurality of capture probes being immobilized on a substrate, the capture probe having a first portion including a sequence capable of hybridizing with a nucleic acid as a detection target, a second portion including a sequence capable of hybridizing with a detection probe labeled with a labeling substance, and a stem portion for forming a double strand.
(6) A nucleic acid detection kit according to one embodiment of the present invention includes: a capture probe; and a detection probe, in which the capture probe includes a first portion including a sequence capable of hybridizing with a nucleic acid as a detection target, a second portion including a sequence capable of hybridizing with the detection probe, and a stem portion for forming a double strand, and the detection probe is labeled with a labeling substance.
(7) A nucleic-acid-immobilized solid phase according to one embodiment of the present invention in which a nucleic acid-detection probe-capture probe complex is immobilized on a solid phase, the nucleic acid-detection probe-capture probe complex is formed by linking a detection probe and a capture probe via a nucleic acid, the capture probe includes a first portion including a sequence capable of hybridizing with the nucleic acid as a detection target, a second portion including a sequence capable of hybridizing with the detection probe, and a stem portion for forming a double strand, and the detection probe and the nucleic acid, and the nucleic acid and the capture probe are linked to each other.
(8) A fluid device according to one embodiment of the present invention includes:
   a nucleic acid detection portion having a solid phase formed by a capture probe being immobilized, in which the capture probe includes a first portion including a sequence capable of hybridizing with a nucleic acid as a detection target, a second portion including a sequence capable of hybridizing with a detection probe labeled with a labeling substance, and a stem portion for forming a double strand.

### Brief Description of Drawings

FIG. 1 is a schematic view of one aspect of a method for detecting a nucleic acid according to an embodiment.
FIG. 2 is a schematic view of one aspect of the method for detecting a nucleic acid according to the embodiment.
FIG. 3 is a schematic view of one aspect of the method for detecting a nucleic acid according to the embodiment.
FIG. 4 is a schematic view of one aspect of the method for detecting a nucleic acid according to the embodiment.
FIG. 5 is a schematic view illustrating one aspect of the method for detecting a nucleic acid according to the embodiment.
FIG. 6 is a schematic view of one aspect of a detecting apparatus having a microarray according to the embodiment.
FIG. 7 is a schematic view illustrating one aspect of the method for detecting a nucleic acid according to the embodiment.
FIG. 8 is a schematic view illustrating one aspect of the method for detecting a nucleic acid according to the embodiment.
FIG. 9 is a schematic view of one aspect of a fluid device according to the embodiment.
FIG. 10 is a detection result of a miRNA according to Examples.
FIG. 11 is a detection result of a miRNA according to Examples.

### Description of Embodiments

### <Method for detecting a nucleic acid>

A nucleic acid as a detection target is not particularly limited and examples thereof include a short RNA such as a miRNA and a short mRNA in which transcription is stopped midway, and a nucleic acid aptamer. For example, the nucleic acid as a detection target is a miRNA which exists in various types within a living body and is involved in the regulation of gene expression.

For example, preferred examples of a solid phase in which a capture probe is immobilized include a substrate or a carrier. Examples of the solid phase carrier include magnetic beads, gold nanoparticles, agarose beads, and plastic beads. Examples of the solid phase substrate include a glass substrate, a silicon substrate, a plastic substrate, and a metal substrate.

### [First embodiment]

The method for detecting a nucleic acid of the embodiment includes the following steps:
(a) a step of bringing a solution including a miRNA and a detection probe labeled with a labeling substance into contact with a substrate on which a capture probe is immobilized, the capture probe having a first portion including a sequence capable of hybridizing with the miRNA, a second portion including a sequence capable of hybridizing with the detection probe, and a stem portion for forming a double strand and having a loop structure;
(b) a step of hybridizing the miRNA with the first portion, hybridizing the detection probe with the second portion, and forming a miRNA-detection probe-capture probe complex on the substrate;
(c) a step of ligating the miRNA and the detection probe and ligating the miRNA and the capture probe in a state where the miRNA is hybridized with the first portion and the detection probe is hybridized with the second portion;
(d) a step of eliminating binding between the capture probe and the detection probe not ligated with the miRNA; and
(e) a step of detecting the labeling substance in the miRNA-detection probe-capture probe complex in which the miRNA and the detection probe are ligated and which is formed on the substrate.

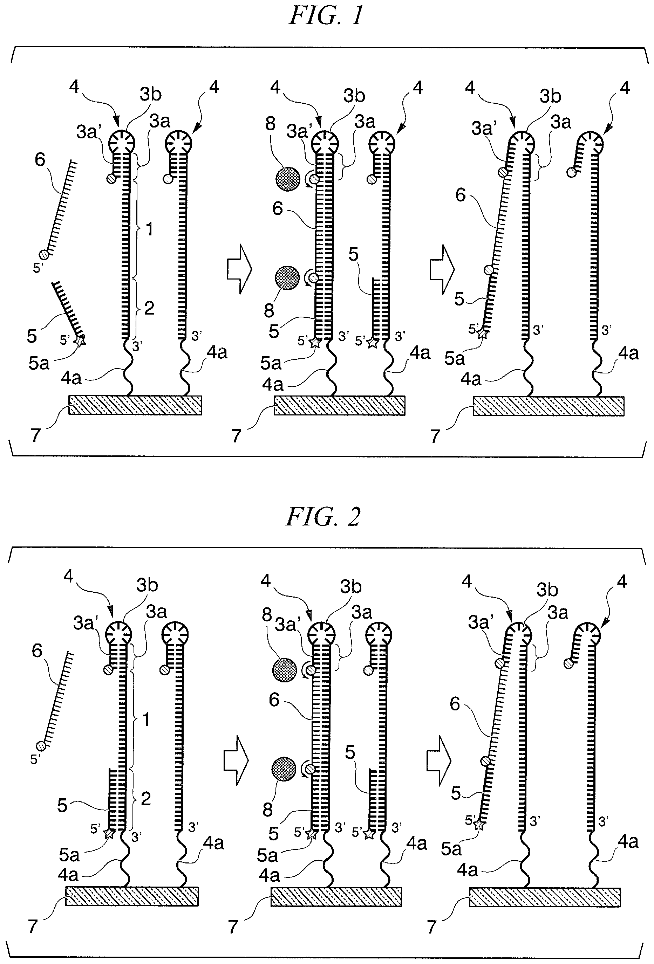

Hereinafter, each step according to the embodiment will be described with reference to FIG. 1.

The step (a) is a step of bringing a solution including a miRNA and a detection probe labeled with a labeling substance into contact with a substrate on which a capture probe is immobilized, the capture probe having a first portion including a sequence capable of hybridizing with the miRNA, a second portion including a sequence capable of hybridizing with the detection probe, and a stem portion for forming a double strand and having a loop structure (hereinafter, referred to as a stem-loop structure).

FIG. 1 is schematic view of one aspect of the method for detecting a nucleic acid according to the embodiment. As illustrated in FIG. 1, a capture probe 4 includes a first portion 1, a second portion 2, stem portions 3a and 3a' for forming a double strand and having a loop structure 3b, and a spacer 4a. The first portion includes a sequence capable of hybridizing with a miRNA 6. The second portion includes a sequence capable of hybridizing with a detection probe 5.

From a viewpoint of detecting the miRNA 6 highly precisely, the second portion of the capture probe 4 preferably does not include a sequence that is complementary to the sequence of the miRNA 6 so as not to be hybridized with the miRNA 6. In the same manner, the second portion of the capture probe 4 preferably does not include a sequence that is complementary to the sequence of the first portion 1 so as not to be hybridized with the first portion 1 of the capture probe 4 itself.

The length of the first portion 1 (for example, the number of bases) may be set to the same length (for example, the number of bases) as the length of the miRNA 6, in consideration of the miRNA 6 being capable of being hybridized with the first portion 1 of the capture probe 4.

The length of the second portion 2 (for example, the number of bases) is not particularly limited, and may be about 10 to 30 bases from a viewpoint of (1) the detection probe 5 being preferably capable of being hybridized with the second portion 2 at a temperature close to the optimal temperature (37°C) of T4 DNA ligase and (2) the second portion 2 preferably having a sequence specificity. The aforementioned two points are affected by the GC content of the second portion 2. In a case where the length of the second portion 2 is set to about 5 to 10 bases, a stabilization effect of the double strand structure of the nucleic acid is more remarkably exhibited by a stacking effect, and accordingly, it is considered that the detection probe 5 is hybridized with the second portion 2 mainly using the effect. However, from a viewpoint of increasing the sequence specificity to hybridize the detection probe 5 more precisely for a short period of time, the length of the second portion 2 is preferably 10 to 30 bases and more preferably 18 to 25 bases. In addition, for example, the number of bases of the second portion 2 may be the number of bases larger than the number of bases in which the stacking effect is mainly exhibited.

The length of the stem portions 3a and 3a' (for example, the number of bases) is not particularly limited and may be about 8 to 15 bases, which is roughly estimated, in consideration of the length in which a double strand can be stably formed. In the stem portions 3 a and 3 a', the entire stem portions do not need to form a base pair of a double strand, but if a case of the embodiment is exemplified, since the stem portions 3a and 3a' are adjacent to the first portion 1 as illustrated in FIG. 1 and the miRNA 6 is hybridized with the first portion 1, at least one base adjacent to the first portion 1 of the stem portions 3a and 3a' preferably forms a base pair.

The detection probe 5 is labeled with a labeling substance 5a. The arrangement of the labeling substance 5a in the detection probe 5 is not particularly limited, and from a viewpoint of steric hindrance at the time of forming a miRNA 6-detection probe 5-capture probe 4 complex described below and not hindering ligase to approach the miRNA 6-detection probe 5-capture probe 4 complex described below, the labeling substance 5a is preferably not bonded to the end adjacent to the miRNA, preferably bonded to a side opposite to the side of the end adjacent to the miRNA, and preferably bonded to an end on the side opposite to the side of the end adjacent to the miRNA. In the embodiment, in the detection probe 5, the end opposite to the end adjacent to the miRNA is a 5' end of the detection probe 5, and as illustrated in FIG. 1, the labeling substance 5a is bonded to the side of the 5' end of the detection probe 5.

Examples of the labeling substance include a fluorescent pigment, a fluorescent bead, a quantum dot, biotin, an antibody, an antigen, an energy-absorbing substance, a radioisotope, a chemiluminescent body, and an enzyme.

Examples of the fluorescent pigment include FAM (carboxyfluorescein), JOE (6-carboxy-4',5'-dichloro 2',7'-dimethoxyfluorescein), FITC (fluorescein isothiocyanate), TET (tetrachlorofluorescein), HEX(5'-hexachloro-fluorescein-CE phospholoamidite), Cy3, Cy5, Alexa568, and Alexa647.

Since the miRNA included in the total RNA exists in an extremely small amount, it is difficult to label the miRNA highly effectively. Meanwhile, in the embodiment, the miRNA can be quantified in a highly sensitive manner in order to use the detection probe labeled in advance.

In a case where the capture probe 4 is labeled with the labeling substance, a combination of the labeling substance which labels the capture probe 4 and the labeling substance which labels the detection probe 5 may be a combination that may not cause FRET (Fluorescence (Foerster) Resonance Energy Transfer) or a combination that may cause FRET.

From a viewpoint of obtaining a different FRET efficiency caused by the sequence or the length of the target miRNA, a combination that may not cause FRET is preferable. Even in a case where a combination of the labeling substance that may cause FRET is used, it is possible to design such that FRET does not occur between the capture probe and the detection probe, for example, labeling the terminal close to the substrate of the capture probe with FAM and labeling a loop portion the farthest from the substrate of the detection probe with Alexa647.

Meanwhile, from a viewpoint of being able to distinguish a case where the detection probe is linked to the capture probe and a case where the detection probe is adsorbed to the substrate, a combination that may cause FRET is preferable.

As the combination of the labeling substance that may cause FRET, a fluorescent pigment having an excitation wavelength close to 490 nm (for example, FITC, Rhodamine green, Alexa (registered trademark) fluor 488, BODIPY FL, or the like) and a fluorescent pigment having an excitation wavelength close to 540 nm (for example, TAMRA, Tetramethyl Rhodamine, Cy3), or a combination of the fluorescent pigment having an excitation wavelength close to 540 nm and a fluorescent pigment having an excitation wavelength close to 630 nm (for example, Cy5, or the like) is preferable.

In order for the capture probe 4 immobilized to a substrate 7 to be hybridized with the miRNA 6, since a molecular freedom is necessary, the capture probe 4 preferably has a spacer 4a at the 3' end bonded to the substrate 7. In addition, in order for ligase to easily approach the miRNA 6-detection probe 5-capture probe 4 complex described below, the capture probe 4 preferably has a spacer 4a at the 3' end bonded to the substrate 7.

The length of the spacer 4a (for example, the number of bases) is not particularly limited, and preferably 3 to 50 bases and more preferably 5 to 25 bases. However, the base to be used for the spacer can be replaced by a linker such as PEG, which has the same length and softness as the base. In this case, the number of bases to be used for the spacer 4a may be 0.

The sample containing the miRNA (nucleic acid) is not particularly limited, and in a case where the method for detecting a nucleic acid of the embodiment is used for diagnosing cancer, the sample is preferably obtained by extracting a nucleic acid from a sample such as blood, lymph fluid, cerebrospinal fluid, seminal fluid, saliva, and urine of a subject, for example, a patient in which the onset of cancer is confirmed, a patient in which the onset of cancer is suspected, or a patient in which cancer is treated. The extraction of the nucleic acid from the above sample can be conducted according to a usual method, for example, using trizol, or the like, but a method for extracting short RNA is preferably used.

As described above, since the amount of a miRNA in blood is extremely small at 0.01% by mass in the total RNA, a miRNA concentrated by a fraction from the total RNA extracted from the sample may be used as a nucleic acid sample, but in the embodiment, since the miRNA itself as a detection target does not need to be labeled with a labeling substance, a miRNA in which a fraction operation has not been conducted may be used as a nucleic acid sample.

Examples of the substrate 7 used for immobilizing the capture probe 4 include a glass substrate, a silicon substrate, a plastic substrate, and a metal substrate. As a method for immobilizing the capture probe 4 on the substrate 7, a method for immobilizing a probe on a substrate in a highly dense manner using photolithography, or a method for immobilizing a probe on a glass substrate by spotting is exemplified.

In a case of using photolithography, the capture probe 4 may be synthesized on the substrate 7.

In a case of immobilizing the capture probe 4 by spotting, it is preferable to provide a solid phase binding site to the capture probe 4 and provide a solid phase binding portion recognition site to the substrate 7.

Examples of a combination of the solid phase binding site and the solid phase binding portion recognition site include a combination of the solid phase binding site provided by modifying the capture probe 4 with a functional group such as an amino group, a formyl group, a SH group, and a succinimidyl ester group, a solid phase binding portion recognition site provided by surface treating the substrate 7 with a silane coupling agent having an amino group, a formyl group, an epoxy group, or a maleimide group; and a combination using gold-thiol bonding.

In addition, as another method for immobilizing a capture probe by spotting, a method is exemplified in which a capture probe having a silanol group is discharged on a glass substrate and arrayed to be covalently bonded to the substrate by a silane coupling reaction.

The detection probe 5 may be ligated with a nucleic acid. In a case of using T4 DNA ligase for ligation, the detection probe 5 is preferably RNA and may include an artificial nucleic acid such as a 2'-O-methyl RNA, a LNA (Locked Nucleic Acid), and a BNA (Bridged Nucleic Acid), if the artificial nucleic acid has the same function as the nucleic acid. The detection probe 5 preferably includes a 2'-O-methyl RNA, a LNA, or a BNA from a viewpoint of having high affinity with a target miRNA, being unlikely to be recognized by a DNA nuclease or a RNA nuclease, and being able to become a substrate of a DNA ligase such as T4 DNA ligase, compared to the RNA. However, the 3' end of the detection probe 5 (the end at the side adjacent to the nucleic acid) is preferably a RNA because of properties of the T4 DNA ligase.

Either the capture probe 4 or the detection probe 5 preferably includes a 2'-O-methyl RNA, a LNA, or a BNA, and both the capture probe 4 and the detection probe 5 more preferably include a 2'-O-methyl RNA, a LNA, or a BNA.

The step (b) is a step of hybridizing the miRNA with the first portion, hybridizing the detection probe with the second portion, and forming a miRNA-detection probe-capture probe complex on the substrate.

In the step (b), since the miRNA is likely to form a steric structure, it is preferable to thermally denaturalize the miRNA by incubation at a temperature of 95°C for about 5 minutes to cause the miRNA to be in a state that is easily hybridized with a probe.

The hybridization is not particularly limited and can be conducted under common conditions of a temperature, pH, a salt concentration, and a buffer solution, in consideration of a Tm value of each probe. However, from a viewpoint of quantifying the miRNA in a highly precise manner, it is preferable to conduct hybridization under a stringent condition.

In addition, the term "capable of hybridizing" in the present invention and the present specification includes that at least a part of the target nucleic acid (the target miRNA) or the detection probe used in the present invention is hybridized with the capture probe to form a complex complementarity. As the "stringent condition", a condition disclosed in Molecular Cloning-A LABORATORY MANUAL THIRD EDITION (Sambrook et al., Cold Spring Harbor Laboratory Press) is exemplified, and a condition in which a temperature condition is 30°C (a temperature condition about 5°C to 10°C higher than the Tm of the sequence of the probe) and a condition in which a salt concentration condition is less than 1 M is exemplified.

As illustrated in FIG. 1, the capture probe 4 is immobilized on the substrate 7 at the 3' side, the capture probe 4 is disposed in the order of the spacer 4a, the second portion 2, the first portion 1, the stem portions 3a and 3a', and the loop structure 3b from the side of the substrate 7.

The number of the detection probe 5 existing in the solution is commonly set to be larger than the number of the miRNA 6 in order to enhance efficiency of hybridization, and in this case, it is more likely that the detection probe 5 has been already hybridized with the second portion 2 of the capture probe 4, before the miRNA 6 is hybridized with the first portion 1 of the capture probe 4.

Here, in the embodiment, since the first portion 1 is positioned between the second portion 2 and the stem portions 3 a and 3a', the miRNA 6 of the detection probe causes the first portion 1 to be exposed between the double strand of the stem portions 3a and 3 a' and the double strand formed by the second portion 2 and the detection probe 5.

In this way, both terminals of the first portion 1 are in contact with the double strand and the first portion 1 is surrounded by the double strand, and accordingly, it is possible to prevent the miRNA having a longer length than that of the first portion 1 surrounded by the double strand from being bound to the first portion 1. For example, it is possible to prevent a pre-miRNA (a precursor of the miRNA) from being bound to the first portion 1 and detect only a mature miRNA more accurately. In addition, it is possible to hybridize the miRNA 6 with the first portion 1 more effectively by a stacking effect.

The step (c) is a step of ligating the miRNA and the capture probe and ligating the miRNA and the detection probe in a state where the miRNA is hybridized with the first portion and the detection probe is hybridized with the second portion.

As illustrated in FIG. 1, the 5' end of the capture probe 4 and the 3' end of the miRNA 6 are ligated. As the capture probe 4 and the miRNA 6 are ligated, it is possible to prevent the miRNA 6 hybridized with the capture probe 4 from being dissociated from the capture probe 4, and in the step (e), it is possible to prevent the miRNAs 6 from being detected over a plurality of times.

In addition, as illustrated in FIG. 1, the 5' end of the miRNA 6 and the 3' end of the detection probe 5 are ligated. As the detection probe 5 and the miRNA 6 are ligated, the detection probe 5 and the capture probe 4 are bound to each other via the miRNA 6. Thus, the detection probe 5 and the capture probe 4 can be bound to each other only when the miRNA 6 is hybridized with the first portion 1. According to the embodiment, since the detection probe 5 and the capture probe 4 can be bound to each other only when the miRNA 6 is hybridized with the first portion 1, the miRNA can be detected in a highly precise manner.

As an enzyme used for ligation, ligase is exemplified, and as the ligase, T4 DNA ligase, T4 RNA ligase 2, and T4 RNA ligase 1 are exemplified.

In the step (c), the 5' end of the capture probe 4 and the 3' end of the detection probe 5 may be ligated, which is an unintended reaction. It is considered that such an unintended ligation reaction between the capture probe 4 and the detection probe 5 is caused by a ligation activity between single-strand nucleic acids. Thus, it is preferable that a ligation reaction of the miRNA and the detection probe on the capture probe be preferentially performed rather than the above ligation reaction. At this point, since the T4 DNA ligase exhibits an activity in a double-strand-dependent manner, a problem is unlikely to occur in the ligation of the 5' end of the capture probe 4 and the 3' end of the detection probe. Thus, the T4 DNA ligase is preferable as the enzyme to be used for ligation. FIG. 1 illustrates a state of the ligation of the capture probe 4 and the miRNA 6, and the ligation of the miRNA 6 and the detection probe 5 by T4 DNA ligase 8.

The step (b) and the step (c) may be performed at the same time. That is, hybridization and ligation may be performed at the same time.

In the embodiment, at the time of the ligation in the step (c), it is preferable to phosphorylate the 5' end of the capture probe 4 in advance using an enzyme such as T4 polynucleotide kinase. The miRNA originated from a living body has a phosphate group at the 5' end.

The step (d) is a step of eliminating binding between the capture probe and the detection probe not ligated with the miRNA.

For example, in the embodiment, the step (d) is a step of eliminating binding between the capture probe and the detection probe not ligated with the miRNA and not linked to the capture probe via the miRNA. In addition, for example, as illustrated in FIG. 1, the step (d) is a step of eliminating binding between the capture probe 4 and the detection probe 5 not ligated with the miRNA 6 among the plurality of detection probes 5 immobilized on the substrate 7. In addition, for example, among the detection probe 5 not ligated with the miRNA 6 and the detection probe 5 ligated with the miRNA 6 in the plurality of detection probes 5 immobilized on the substrate 7, binding between the detection probe 5 not ligated with the miRNA 6 and the capture probe 4 is eliminated in the step (d).

The detection probe 5 can be hybridized with the second portion 2 of the capture probe 4 even in a case where the miRNA 6 is not hybridized with the first portion 1. Thus, inclusion of the step (d) for eliminating binding between the capture probe and the detection probe not ligated with the miRNA in the method for detecting a nucleic acid of the embodiment enables detection of the existence of the miRNA 6 in a highly precise manner.

In the embodiment, as a method for eliminating binding between the capture probe and the detection probe not ligated with the miRNA, a method is exemplified which places the capture probe under a condition where hybridization between the miRNA and the capture probe, and the detection probe and the capture probe can be eliminated. In addition, a method is exemplified which places the capture probe under a condition where hydrogen bonding that forms a double strand between the miRNA and the capture probe, and the detection probe and the capture probe can be cut. At this time, the above condition is preferably a condition in which hydrogen bonding that forms a double strand of the nucleic acid can be cut and phosphodiester bonding (covalent bonding) between nucleotides are not eliminated. As the condition, a condition that is commonly used in a case of denaturing the nucleic acid can be broadly adopted. For example, a liquid having a temperature higher than the Tm value of the sequence of the detection probe (the substrate is preferably cleansed after denaturalization of the nucleic acid such that the detection probe is not bound again) or various reagent solutions that can denaturalize the nucleic acid of the double strand to a single strand can be exemplified. As the reagent, an agent that is commonly used as a denaturing agent of the nucleic acid can be broadly adopted and examples thereof include alkali such as sodium hydroxide, a surfactant such as sodium dodecyl sulfate (SDS), urea, formamide, and formaldehyde. These conditions and reagents may be used alone or a plurality thereof may be used in combination. Among these, from a viewpoint of being capable of denaturing the nucleic acid without decomposing the RNA, a method for bringing formamide having a final concentration of 20% to 80% (v/v) and the SDS solution having a final concentration of 0.05% to 10% (w/v) into contact with the substrate can be exemplified.

As illustrated in FIG. 1, if the hydrogen bonding that forms a double strand between the capture probe and the detection probe is cut, the detection probe 5 not ligated with the miRNA 6 is dissociated from the capture probe 4. In contrast, even if the hydrogen bonding that forms a double strand between the capture probe and the detection probe is cut, the detection probe 5 ligated with the miRNA 6 is linked to the capture probe 4 via the miRNA 6 and a state of the miRNA-detection probe-capture probe complex is maintained.

In this way, it is possible to remarkably increase efficiency of eliminating non-specific bonding by placing the capture probe under a condition in which hybridization between the miRNA and the capture probe, and the detection probe and the capture probe can be eliminated.

In a case where a similar miRNA that is not completely complementary to the first portion is bound to the first portion, a step of decomposing phosphodiester bonding of a mismatch region between the similar miRNA and the capture probe may be performed between the step (c) and the step (d), in order to prevent the similar miRNA from being detected.

As a method for decomposing phosphodiester bonding of a mismatch region between the similar miRNA and the capture probe, a method disclosed in the United States Patent No. 5891629 "Compositions For Improving Rnase Cleavage Of Base Pair Mismatches In Double-stranded Nucleic Acids" is exemplified and this method is adopted as a part of the present specification. For example, an enzyme having a mismatch region recognition ability and a mismatch region decomposition ability may be used and examples of the enzyme include various RNase such as RNase A, RNase I, and RNase T1.

In the step (d), in order to eliminate the detection probe 5 whose binding to the capture probe 4 is eliminated and the detection probe 5 non-specifically hybridized with the substrate, it is preferable to provide a step of cleansing the substrate 7 after the step (d) or at the same time with the step (d). For cleansing, a method for cleansing the substrate 7 by shaking the substrate 7 in the aforementioned denaturing agent is exemplified.

The step (e) is a step of detecting the labeling substance in the miRNA-detection probe-capture probe complex in which the miRNA and the detection probe are ligated and which is formed on the substrate. Here, the "detecting" includes detecting quantitatively, quantifying, and analyzing.

As described above, since the detection probe 5 is labeled with the labeling substance 5a, the labeling substance 5a in the miRNA 6-detection probe 5-capture probe 4 complex can be detected.

At this time, a standard curve is fabricated using the miRNA in the known amount, which is gradationally diluted, and the standard curve is preferably used. The miRNA 6 in the nucleic acid sample can be quantified by using the detection result.

In the step (e), the method for detecting a labeling substance is not particularly limited, and a method that is commonly used in a case of detecting a nucleic acid can be used, for example, measuring fluorescence intensity of the complex using a nucleic acid microarray automatic detecting apparatus or detecting the complex using an electrochemical detector.

According to the method for detecting the miRNA of the embodiment, it is possible to detect the target miRNA in a highly precise manner.

In addition, according to the method for detecting the miRNA of the embodiment, since the capture probe forming a stem-loop structure is used, a possibility of recognizing a pre-miRNA can be further reduced and the target miRNA can be detected in a highly precise manner.

As another advantage of the method for detecting the miRNA of the embodiment, even in a case where the sequences of the miRNA as a detection target are different from each other, the miRNA can be detected by using a common detection probe. This is because, in the method for detecting the miRNA of the embodiment, since the detection probe and the miRNA are not directly hybridized with each other, the sequence of the detection probe can be freely designed without being influenced by the sequence of the miRNA. By using the common detection probe, it is possible to provide the method for detecting the miRNA at a lower price, compared to a case of using the detection probe with the sequence of the miRNA combined.

### [Second Embodiment]

In the method for detecting a nucleic acid of the [First Embodiment] previously described, a solution including a detection probe and a miRNA is brought into contact with a substrate on which a capture probe is immobilized. In contrast, in the method for detecting a nucleic acid of the second embodiment, a solution including a miRNA is brought into contact with a substrate on which a capture probe is immobilized in a state where the detection probe is hybridized in advance. In addition, in the embodiment, the stem portion has a loop structure.

For example, the method for detecting a nucleic acid of the embodiment includes the following steps:
(a') a step of bringing a solution including a miRNA into contact with a substrate on which a capture probe is immobilized, the capture probe having a first portion including a sequence capable of hybridizing with the miRNA, a second portion including a sequence capable of hybridizing with a detection probe, and a stem portion for forming a double strand and having a loop structure,
   wherein the detection probe labeled with a labeling substance is hybridized with the second portion in advance;
(b') a step of hybridizing the miRNA with the first portion and forming a miRNA-detection probe-capture probe complex on the substrate;
(c) a step of ligating the miRNA and the capture probe ligating the miRNA and the detection probe in a state where the miRNA is hybridized with the first portion and the detection probe is hybridized with the second portion;
(d) a step of eliminating binding between the capture probe and the detection probe not ligated with the miRNA; and
(e) a step of detecting the labeling substance in the miRNA-detection probe-capture probe complex in which the miRNA and the detection probe are ligated and which is formed on the substrate.

Hereinafter, the embodiment is described with reference to FIG. 2. In addition, a description of the common points to the [First Embodiment] previously described is omitted.

The step (a') is a step of bringing a solution including a miRNA into contact with a substrate on which a capture probe is immobilized, the capture probe having a first portion including a sequence capable of hybridizing with the miRNA, a second portion including a sequence capable of hybridizing with a detection probe, and a stem portion for forming a double strand and having a loop structure,
wherein the detection probe labeled with a labeling substance is hybridized with the second portion in advance.

FIG. 2 is a schematic view of one aspect of the method for detecting a nucleic acid according to the embodiment. As illustrated in FIG. 2, the capture probe 4 includes the first portion 1, the second portion 2, the stem portions 3a and 3a' having a loop structure 3b and for forming a double strand, the spacer 4a, and the detection probe 5 hybridized with the second portion 2. The first portion includes a sequence capable of hybridizing with the miRNA 6.

The step (b') is a step of hybridizing the miRNA with the first portion and forming a miRNA-detection probe-capture probe complex on the substrate;

As illustrated in FIG. 2, the capture probe 4 is immobilized on the substrate 7 on the side 3', and the capture probe 4 is disposed in the order of the spacer 4a, the second portion 2 and the detection probe 5, the first portion 1, the stem portions 3a and 3a', and the loop structure 3b from the side of the substrate 7. Since the detection probe 5 labeled with the labeling substance 5a is hybridized with the second portion 2 of the capture probe 4 in advance, the miRNA 6 of the detection probe causes the first portion 1 to be exposed between the double strand of the stem portions 3a and 3a' and the double strand formed by the second portion 2 and the detection probe 5.

According to the method for detecting the miRNA of the embodiment, both terminals of the first portion 1 are in contact with the double strand and the first portion 1 is surrounded by the double strand, and accordingly, it is possible to prevent a pre-miRNA (a precursor of the miRNA) from being bound to the first portion 1 and detect only the miRNA more accurately. In addition, it is possible to hybridize the miRNA 6 with the first portion 1 more effectively by a stacking effect.

In addition, since the capture probe in which the detection probe is hybridized in advance is used, a solution including the capture probe may not be prepared in order for the capture probe to be in contact with the substrate. Thus, an operator may prepare only the nucleic acid as a detection target and can reduce a burden as an executer. Also, convenience of using the method can be enhanced.

Since the capture probe forming a stem-loop structure is used, a possibility of recognizing a pre-miRNA can be further reduced and the target miRNA can be detected in a highly precise manner.

### [Third Embodiment]

In the step (c) of the method for detecting a nucleic acid of the [First Embodiment] previously described, ligation is performed at two sites, which is ligation between the miRNA and the capture probe, and ligation between the miRNA and the detection probe. In contrast, in the third embodiment, ligation between the miRNA and the detection probe is only performed and a linkage of the miRNA and the capture probe is not performed. In addition, the stem portion does not have a loop structure.

For example, the method for detecting a nucleic acid of the embodiment includes the following steps:
(a) a step of bringing a solution including a miRNA and a detection probe labeled with a labeling substance into contact with a substrate on which a capture probe is immobilized, the capture probe having a first portion including a sequence capable of hybridizing with the miRNA, a second portion including a sequence capable of hybridizing with the detection probe, and a stem portion for forming a double strand;
(b) a step of hybridizing the miRNA with the first portion, hybridizing the detection probe with the second portion, and forming a miRNA-detection probe-capture probe complex on the substrate;
(c) a step of ligating the miRNA and the detection probe in a state where the miRNA is hybridized with the first portion and the detection probe is hybridized with the second portion;
(d) a step of eliminating binding between the capture probe and the detection probe not ligated with the miRNA; and
(e) a step of detecting the labeling substance in the miRNA-detection probe-capture probe complex in which the miRNA and the detection probe are ligated and which is formed on the substrate.

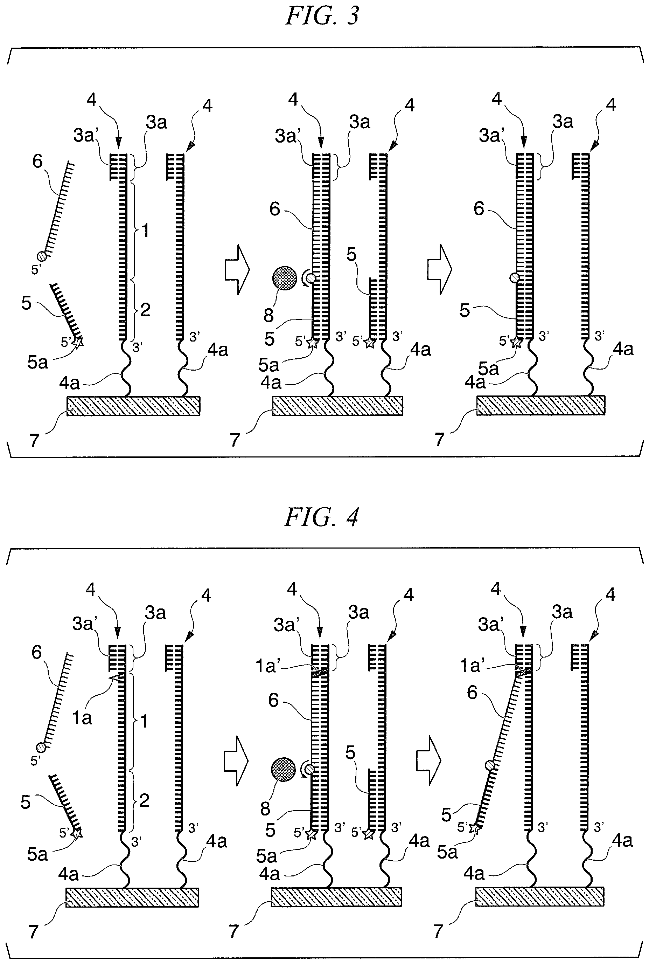

Hereinafter, the embodiment is described with reference to FIG. 3. In addition, a description of the common points to the [First Embodiment] previously described is omitted.

The steps (a), (b), and (e) of the embodiment may be performed in the same manner as the steps (a), (b), and (e) of the [First Embodiment] as illustrated in FIG. 1.

The step (c) is a step of ligating the miRNA and the detection probe in a state where the miRNA is hybridized with the first portion and the detection probe is hybridized with the second portion.

FIG. 3 is a schematic view of one aspect of the method for detecting a nucleic acid according to the embodiment. As illustrated in FIG. 3, the 5' end of the miRNA 6 and the 3' end of the detection probe 5 are ligated. At this time, the 5' end of the capture probe 4 and the 3' end of the miRNA 6 are not ligated.

The step (d) is a step of eliminating binding between the capture probe and the detection probe not ligated with the miRNA.

In the embodiment, as a method thereof, a method for placing the capture probe under a condition in which hybridization between the detection probe and the capture probe is eliminated but the hybridization between the detection probe-miRNA complex and the capture probe is not eliminated is exemplified.

For example, a method for placing the capture probe under a condition in which hydrogen bonding between the detection probe not ligated with the miRNA and the capture probe is eliminated but hydrogen bonding between the detection probe ligated with the miRNA and the capture probe is not eliminated is exemplified. The detection probe ligated with the miRNA has longer polynucleotide by the length of the miRNA, compared to the detection probe not ligated with the miRNA. Due to the difference between the sequence lengths, binding energy generated between the detection probe-miRNA complex and the capture probe is higher than the binding energy generated between the detection probe and the capture probe. As a method for distinguishing the detection probe not ligated with the miRNA and the detection probe ligated with the miRNA using the difference in binding energy, a conventionally known method such as a method for increasing the temperature of a solution gradually may be adopted.

In addition, in order not to eliminate a double strand state of the stem portions 3a and 3a', it is preferable to link between the double strands of the stem portions 3a and 3a'. For example, a photoreactive base derivative described below is disposed in the sequence of the stem portions 3a and 3a' so as to crosslink between the double strands of the stem portions 3a and 3a' (not illustrated), and dissociation between the double strands of the stem portions 3a and 3a' can be prevented.

In this way, even in a case where the capture probe and the miRNA 6 are not ligated, it is possible to detect the existence of the miRNA.

### [Fourth Embodiment]

In the step (c) of the a method for detecting a nucleic acid of the [First Embodiment] previously described, ligation is performed at two sites, which is ligation between the miRNA and the capture probe, and ligation between the miRNA and the detection probe. In contrast, in the fourth embodiment, a linkage between the miRNA and the detection probe is caused by ligation but a linkage between the miRNA and the capture probe is performed by crosslinking between the nucleic acids using a photoreactive base derivative.

For example, the method for detecting a nucleic acid of the embodiment includes the following steps:
(a) a step of bringing a solution including a miRNA and a detection probe labeled with a labeling substance into contact with a substrate on which a capture probe is immobilized, the capture probe having a first portion including a sequence capable of hybridizing with the miRNA, a second portion including a sequence capable of hybridizing with the detection probe, and a stem portion for forming a double strand, and including a first photoreactive base derivative in the sequence of the first portion;
(b) a step of hybridizing the miRNA with the first portion, hybridizing the detection probe with the second portion, and forming a miRNA-detection probe-capture probe complex on the substrate;
(c) a step of ligating the miRNA and the detection probe in a state where the miRNA is hybridized with the first portion and the detection probe is hybridized with the second portion, and crosslinking the first photoreactive base derivative and the miRNA using a first wavelength light;
(d) a step of eliminating binding between the capture probe and the detection probe not ligated with the miRNA; and
(e) a step of detecting the labeling substance in the miRNA-detection probe-capture probe complex in which the miRNA and the detection probe are ligated and which is formed on the substrate.

Hereinafter, the embodiment is described with reference to FIG. 4. In addition, a description of the common points to the [First Embodiment] previously described is omitted.

The step (a) is a step of bringing a solution including a miRNA and a detection probe labeled with a labeling substance into contact with a substrate on which a capture probe is immobilized, the capture probe having a first portion including a sequence capable of hybridizing with the miRNA, a second portion including a sequence capable of hybridizing with the detection probe, and a stem portion for forming a double strand, and including a first photoreactive base derivative in the sequence of the first portion.

FIG. 4 is a schematic view of one aspect of the method for detecting a nucleic acid according to the embodiment. As illustrated in FIG. 4, the capture probe 4 includes the first portion 1, the second portion 2, the stem portions 3a and 3a' for forming a double strand, and a spacer 4a. The first portion includes a sequence capable of hybridizing with the miRNA 6. The second portion includes a sequence capable of hybridizing with the detection probe 5. In the sequence of the first portion, a first photoreactive base derivative 1a is included.

The photoreactive base derivative means a base derivative whose reactivity is activated upon irradiation with light at a predetermined wavelength and which can crosslink an arbitrary target nucleic acid and the detection probe, and an arbitrary nucleic acid and the capture probe. Examples of the photoreactive base derivative include a base added with psoralen, 3-Cyanovinylcarbazole Nucleoside (hereinafter, referred to as ^{CNV}K), 4-thiothymidine a caged product of adenine reactive group (Kobori et al., Bioorg. Med. Chem., 20:5071-5076 (2012)), 3-carbonylvinyl phenol nucleoside (^{CV}P) (Yoshimura et al., Nucleic Acids Symp. Ser., 48:81-82 (2004)), p-carbamoylvinyl phenolnucleoside (p-^{CV}P) (Ami et al., Org. Biomol. Chem., 5: 2583-2586(2007))), 5-carboxyvinyldeoxyuridine (^{CV}U) (Ogasawara et al., ChemBioChem, 6:1756-1760 (2005)), carbazole-tethered 5-carboxyvinyl-2'-deoxyuridine(^{YCV}U) (Fujimoto et al., Org. Lett., 10: 397-400 (2008)), 5-cyanovinyl-1'-α-2'-deoxyuridine (α-^{C}U), (β-^{C}U (Ogino et al., Angew. Chem. Int. Ed.45:7223-7226(2006)), 5-vinyl-2'-deoxyuridine (^{V}U), 5-heptenyl-2'-deoxyuridine (^{H}U), 5-cyclohexyl vinyl-2'-deoxyuridine (^{HV}U), 5-t-butylvinyl-2'-deoxyuridine (^{BuV}U) (Ogino et al., Org. Biomol. Chem., 7:3163-3167 (2009)), ^{BTV}U, ^{PTV}U, ^{MPTV}U, ^{NTV}U (Ami et al., ChemBioChem 9, 2071-2074 (2008)), N³-methyl-5-cyanovinyl-2'-deoxyuridine (^{MCV}U) (Fujimoto et al., Chem. Commun., 3177-3179 (2005)), and 7-deaza-2'-deoxyadenosine (^{VZ}A) (Saito et al., Tetrahedron Lett., 46:97-99 (2005)).

The first wavelength light is not particularly limited as long as the wavelength light can crosslink an arbitrary miRNA and the capture probe.

The step (b) of the embodiment may be performed in the same manner as the step (b) of the [First Embodiment].

The step (c) is a step of ligating the miRNA and the detection probe in a state where the miRNA is hybridized with the first portion and the detection probe is hybridized with the second portion, and crosslinking the first photoreactive base derivative and the miRNA using a first wavelength light;

As illustrated in FIG. 4, the 5' end of the capture probe 4 and the 3' end of the miRNA 6 are ligated. In addition, the substrate 7 is irradiated with the first wavelength light to crosslink the first photoreactive base derivative 1a and the miRNA 6. 1a in FIG 4 represents a photoreactive base derivative not crosslinked with the miRNA 6 and 1a' represents a photoreactive base derivative crosslinked with the miRNA 6.

The step (d) is a step of eliminating binding between the capture probe and the detection probe not ligated with the miRNA.

For example, in the embodiment, the step (d) is a step of eliminating binding between the capture probe and the detection probe not ligated with the miRNA and not linked to the capture probe via the miRNA.

In the embodiment, placing the capture probe under a condition in which hydrogen bonding that forms a double strand between the miRNA and the capture probe, and the detection probe and the capture probe can be cut is exemplified.

As illustrated in FIG. 4, if the hydrogen bonding that forms a double strand between the capture probe 4 and the detection probe5 is cut, the detection probe 5 not ligated with the miRNA 6 is dissociated from the capture probe 4. In contrast, since the detection probe 5 ligated with the miRNA is linked to the capture probe 4 by crosslinking of the miRNA 6 with the photoreactive base derivative 1a', even if the hydrogen bonding that forms a double strand between the capture probe and the detection probe is cut, the detection probe 5 ligated with the miRNA 6 is linked to the capture probe 4 via the miRNA 6 and a state of the miRNA-detection probe-capture probe complex is maintained.

The step (e) of the embodiment may be performed in the same manner as the step (e) of the [First Embodiment].

In this way, by crosslinking the capture probe and the miRNA 6 using the photoreactive base derivative, it is possible to detect existence of the miRNA.

In addition, in the [First Embodiment] previously described, in the step (c), it is recommended to phosphorylate the 5' end of the capture probe 4 in advance in order to ligate the 5' end of the capture probe 4 and the 3' end of the miRNA 6. Meanwhile, in the embodiment, since the capture probe 4 and the miRNA 6 are crosslinked by the photoreactive base derivative, it is not necessary to phosphorylate the 5' end of the capture probe 4 in advance. The miRNA obtained from a living body has a phosphate group at the 5' end. Thus, the embodiment is excellent in that a phosphorylation treatment is not necessary over the entire steps.

In the [Fourth Embodiment] previously described, although the photoreactive base derivative is disposed on the first portion of the capture probe, the photoreactive base derivative may be disposed on other portions in the nucleic acid-detection probe-capture probe complex or may be disposed so as to be included in plural. For example, it is possible to form the double strand of the stem portion stably by disposing the photoreactive base derivative on the stem portion. In the same manner, it is possible to stably form the double strands of the capture probe and the detection probe by disposing the photoreactive base derivative on the second portion of the capture probe or the detection probe.

However, if the step (e) is performed in a state where the capture probe and the detection probe are crosslinked by the photoreactive base derivative, it is difficult to distinguish whether the miRNA exists or not. In this case, the photoreactive base derivative is preferably a reversible photo-coupling base. The aforementioned problem can be solved by making the photoreactive base derivative be disposed on the second portion of the capture probe or the detection probe be a reversible photo-coupling base.

The reversible photo-coupling base is a photo-coupling base that can reversibly generate photo-dissociation and photo-coupling to the nucleic acid, and the reversible photo-coupling base includes a base that is reversibly photo-coupled and photo-cleaved by being irradiated with wavelength light different from the wavelength light used in the photo-coupling.

If the method for detecting a nucleic acid using a reversible crosslinking reaction is described using the case of [Second Embodiment] previously described as an example, first, the reversible photo-coupling base is disposed on the second portion of the capture probe and the capture probe is hybridized with the second portion, and accordingly, the first wavelength light for photo-coupling is radiated to crosslink the capture probe and the detection probe. Thereafter, before the step (d), a second wavelength light is radiated to eliminate crosslinking with the capture probe and the detection probe. The second wavelength light is wavelength light that can cleave the crosslinking of the capture probe and the detection probe.

As one example of a reversible photo-coupling base derivative, a base added with psoralen, ^{CNV}K, ^{CV}U, ^{YCV}U, α-^{C}U, β-^{C}U, ^{V}U, ^{H}U, ^{HV}U, ^{BuV}U, and ^{VZ}A are exemplified.

In addition, in a case where ^{CNV}K is used as one example of the reversible photo-coupling base, an efficient crosslinking is possible for a short period of time. If a case of using ^{CNV}K as the photoreactive base derivative is described as one example, the first wavelength light is light at a wavelength of 340 nm or more. As one example, by radiating the light at a wavelength of 340 to 380 nm, an atom configuring a pyrimidine base in the miRNA 6 which forms a base pair with a purine base adjacent to the side 5' of ^{CNV}K and an atom configuring ^{CNV}K form a crosslinking structure. The second wavelength light is light at a wavelength of less than 350 nm. As one example, by radiating the light at a wavelength of 280 to 345 nm, the crosslinking is eliminated. The first wavelength and the second wavelength may be partially overlapped in the wavelength.

The use of ^{CNV}K and radiation of the light at a predetermined wavelength for a short period of time (for example, 30 seconds) can obtain high crosslinking efficiency and does not damage the nucleic acid as a radiation target. In addition, the ^{CNV}K is excellent in that a reversible crosslinking reaction is efficiently possible.

### [Fifth Embodiment]

The method for detecting a nucleic acid of the Fifth Embodiment, in addition to the step (a) to the step (e), further includes a step (f) of synthesizing a nucleic acid using the miRNA in the miRNA-detection probe-capture probe complex in which the miRNA and the detection probe are ligated and which is formed on the substrate as a template. As the step (a) to the step (e), the step (a) to step (e) in the [First Embodiment], or the step (a') to the step (e) in the [Second Embodiment] are preferably exemplified.

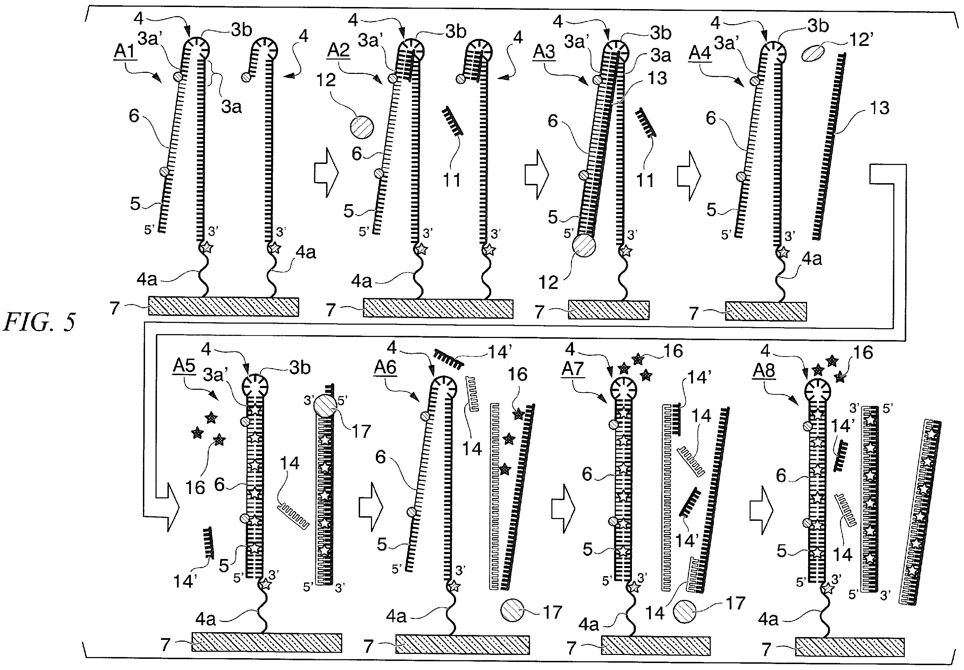

Hereinafter, the embodiment is described with reference to FIGS. 5 to 7.

FIG. 5 is a schematic view illustrating one aspect of the step (f) of the method for detecting a nucleic acid according to the embodiment. First, the step (a) to the step (e) are performed and the miRNA 6-detection probe 5-capture probe 4 complex is formed (FIG. 5, (A1)). Next, a reverse transcription of the sequence including the miRNA 6 and the detection probe 5 is performed by a reverse transcriptase 12 using a RT primer 11 having a base sequence capable of hybridizing with the stem portion 3a' to obtain a cDNA 13 (FIG. 5, (A2) to (A3)). Since the cDNA is not obtained from the capture probe not ligated with the miRNA, the cDNA is synthesized in the amount corresponding to the amount of the miRNA. Thereafter, the reverse transcriptase is thermally denatured by performing a thermal treatment and the cDNA 13 is dissociated from the miRNA 6-detection probe 5-capture probe 4 complex (FIG. 5, (A4)).

Next, a synthesis of the nucleic acid is performed using the aforementioned cDNA as a template. As one example, the following is exemplified in which an amplification of the nucleic acid by a DNA polymerase 17 is performed using a primer set including a first PCR primer 14 having a base sequence capable of hybridizing with the second portion and a second PCR primer 14' having a base sequence capable of hybridizing with the stem portion 3a'. Each of the length of the first PCR primer and the second PCR primer is preferably 10 to 40 bases and more preferably 20 to 30 bases, in the same manner as a common primer.

Examples of an amplification method of a region including an insertion sequence 5f include, in addition to PCR (Polymerase Chain Reaction), conventionally known methods such as LAMP (Loop-Mediated Isothermal Amplification), NASBA (Nucleic Acid Sequence Based Amplification), ICAN (Isothermal and Chimerical primer-initiated Amplification of Nucleic acids), TRC (Transcription Reverse-Transcription Concerted), SDA (Strand Displacement Amplification), TMA (Transcription Mediated Amplification), SMAP (SMart Amplification Process), RPA (Recombines polymerase amplification), and HDA (Helicase-dependent amplification).

The method for detecting a nucleic acid of the embodiment includes a step of detecting an amplification product obtained in a step (g) and the step (f).

The step (g) may be performed after the step (f) or may be performed at the same time (real-time) with the step (f).

For detection of the amplification product, detection by labeling the amplification product itself is exemplified. Examples of the labeling substance used in labeling the amplification product include a fluorescent pigment, a fluorescent bead, a quantum dot, biotin, an antibody, an antigen, an energy-absorbing substance, radioisotope, chemiluminescent body, and an enzyme.

Among these, as the labeling substance, the fluorescent pigment is preferable and an intercalator is more preferable. The intercalator is not particularly limited as long as the intercalator is a substance which emits fluorescent light by intercalating a double strand DNA, which can be detected by fluorescent light or UV. Examples thereof include ethidium bromide, acridine orange, SYBER Green, and SYBER Gold. When the synthesis of the nucleic acid is performed by using the cDNA as a template, by adding these intercalators 16 to a reaction solution for the nucleic acid synthesis (FIG. 5, (A5) to (A8)), or adding a fluorescence reagent to a buffer solution for electrophoresis in a trace amount, the amplification product and the fluorescence reagent are intercalated so as to be able to detect fluorescent light.

Since the sequence of the miRNA is amplified by performing the step (f), it is possible to detect the miRNA which has not been detected by performing the step (a) to the step (e). In particular, by performing the step (g) and the step (f) at the same time (real-time), a dynamic range is drastically enhanced and a detection lower limit concentration of the nucleic acid is improved, compared to a method for comparing a signal intensity by detecting a labeling substance only in the step (e).

In addition, quantification of the miRNA is also possible by performing the step (f).

For example, in a case where a synthesis of the nucleic acid is performed by using the same or the common primer set, it is estimated that synthesis efficiency of the nucleic acid is maintained at the same level even if the sequence of the nucleic acid is different. Thus, by performing the synthesis of the nucleic acid using the miRNA in the miRNA-detection probe-capture probe complex as a template, due to this, it is possible to obtain the amplification product in the amount corresponding to the amount of the detection probe-miRNA-capture probe complex.

As a microarray used for the method for detecting a nucleic acid of the embodiment, a microarray is exemplified in which a spot where a plurality of the capture probes is collected is disposed within a well formed by the substrate and a spacer provided on the substrate, and the capture probe is immobilized on the substrate within the well or the spacer. The capture probe has the first portion including a sequence capable of hybridizing with the nucleic acid as a detection target, the second portion including a sequence capable of hybridizing with the detection probe labeled with the labeling substance, and the stem portion for forming a double strand.

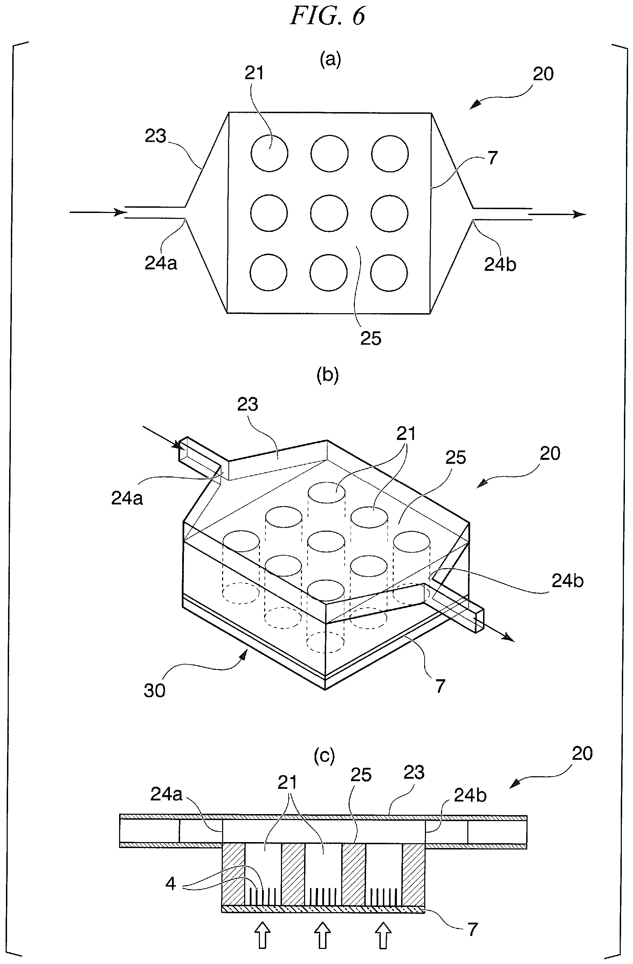

FIG. 6 is a schematic view of one aspect of a detecting apparatus having the microarrayused in the method for detecting a nucleic acid according to the embodiment.

FIG. 6(a) is a plane view illustrating one aspect of the detecting apparatus 20. A spot where a plurality of the capture probes 4 is collected is formed on the substrate 7. The substrate 7 is housed in a housing 23 and a reagent inlet port 24a and a reagent outlet port 24b are provided on a wall of the housing 23. A distance between each well 21 is preferably set to a distance in which signals emitted from the spot are not interfered to each other.

FIG. 6(b) is a perspective view illustrating one aspect of the detecting apparatus 20. The shape of the well 21 is not particularly limited and examples of the shape include a columnar shape, a conical shape, and a polygonal columnar shape. In the embodiment, the shape of the well is a columnar shape as illustrated in FIG. 6(b). The diameter of the well can be set to about 0.1 µm to 1 mm as one example. By providing the spacer on the substrate, the reaction solution for the nucleic acid synthesis is separated for each spot and an independent reaction space can be formed for each spot. Thus, the nucleic acid can be detected for each spot without the amplification product originated from each spot being mixed in the solution. In addition, in a case where the spacer is configured by a shielding material, interference between the signals emitted from the spot can be reduced by the spacer. A heater unit 30 for controlling an amplification temperature of the nucleic acid may be disposed in the lower portion of the substrate.

FIG. 6(c) is a sectional view illustrating one aspect of the detecting apparatus 20. The capture probe 4 is immobilized on the substrate within the well 21. As illustrated in FIG. 6(c), a gap is provided between the upper surface of the spacer 25 and the wall surface of the housing 23. The gap can be used as a space for delivering a reagent to each well.

In addition, in the embodiment, an aspect is exemplified in which the capture probe is disposed on the substrate within the well formed by the spacer provided on the substrate and the substrate, but a concave (well) may be formed on the substrate itself.

In addition, in the embodiment, the capture probe is exemplified which has the first portion including a sequence capable of hybridizing with the nucleic acid as a detection target, the second portion including a sequence capable of hybridizing with the detection probe labeled with the labeling substance, and the stem portion for forming a double strand. However, the capture probe may be a probe as long as the probe has a sequence capable of hybridizing with at least a part of the nucleic acid as a detection target in order to capture the nucleic acid as a detection target. In this case, the method for detecting a nucleic acid includes a step of synthesizing the nucleic acid using the nucleic acid as a detection target as a template, after a step of linking the nucleic acid as a detection target captured by the capture probe to the capture probe, or a step of linking the nucleic acid as a detection target captured by the capture probe to the capture probe via an arbitrary substance. Here, as one example of linking the nucleic acid to the capture probe, ligation by the ligase described in the aforementioned respective embodiments and crosslinking the bases upon irradiation the photoreactive base derivative with light are exemplified. Examples of the arbitrary substance include oligonucleoside having an arbitrary sequence. The oligonucleoside preferably has a stem-loop structure. The stem-loop structure may be a part of the capture probe.

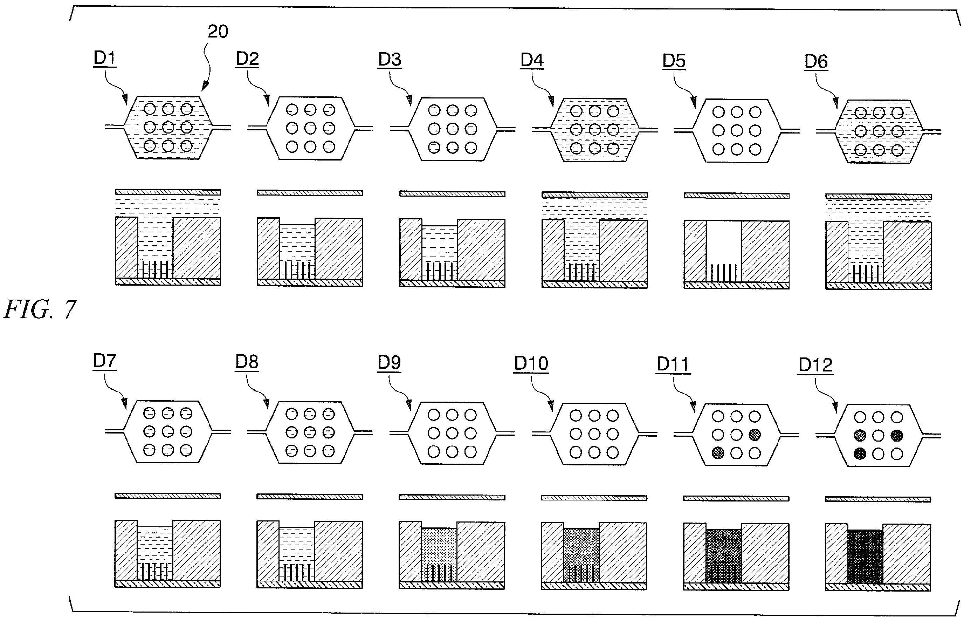

FIG. 7 is a schematic view illustrating the order of the step (f) and the step (g) in the method for detecting a nucleic acid of the embodiment. First, a solution including the miRNA as a detection target, the detection probe, and the ligase is introduced into the detecting apparatus 20 (FIG. 7, (D1)). Thereafter, the extra solution in the gap between the well and the upper portion of the spacer is eliminated, the miRNA is hybridized with capture probe, the detection probe is hybridized with the capture probe, the nucleic acid-detection probe-capture probe complex is formed on the substrate, and the miRNA and the detection probe are ligated (FIG. 7, (D2) to (D3)). Subsequently, a denaturing agent of the nucleic acid is introduced into the detecting apparatus 20 to eliminate binding between the capture probe and the detection probe not ligated with the miRNA, and then a cleansing solution is introduced into the detecting apparatus 20 and the cleansing solution is eliminated (FIG. 7, (D4) to (D5)). By this series of procedures, a miRNA-detection probe-capture probe complex in which the miRNA and the detection probe are ligated or the capture probe which has not captured the miRNA remains on the substrate.

Thereafter, a reaction solution including a reverse transcriptase, a DNA polymerase, a primer, and an intercalator is introduced into the detecting apparatus 20 and the extra solution in the gap between the well and the upper portion of the spacer is eliminated (FIG. 7, (D6) to (D7)). The heater unit is operated to perform a reverse transcription reaction and PCR (FIG. 7, (D8) to (D12)). (D9) to (D12) in FIG. 7 illustrate an aspect in which a fluorescent signal is increased by the nucleic acid synthesis using the cDNA as a template.

### (Plurality of types of capture probes/Plurality of types of detection probes)

One common advantage of the aforementioned method for detecting a nucleic acid exemplified in each embodiment is that since the detection probe and the miRNA are not directly hybridized with each other, the sequence of the detection probe can be freely designed without being influenced by the sequence of the miRNA and the common detection probe can be sued for detecting a different type of the miRNA.

However, the above is not to disturb the use of the plurality of types of capture probes having different sequences of the second portion and the use of the plurality of types of detection probes corresponding thereto.

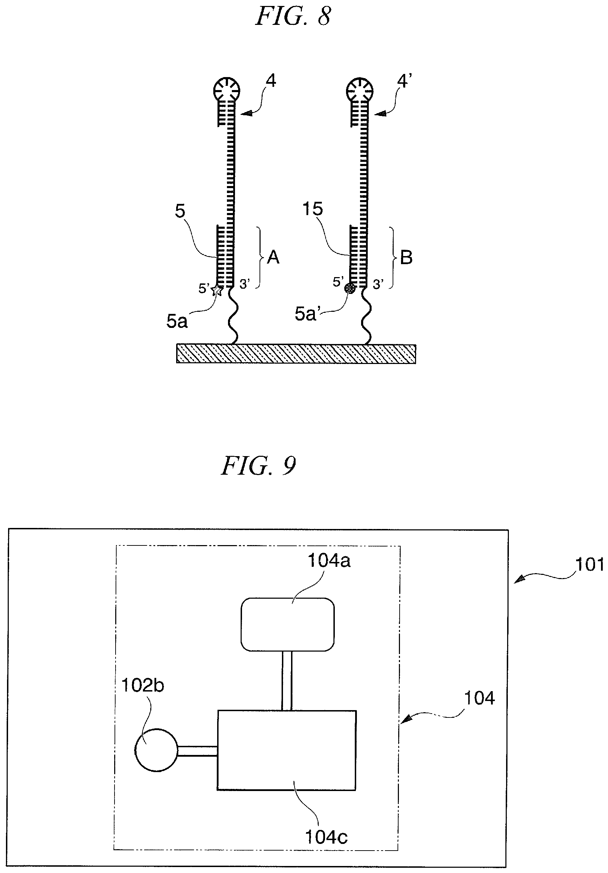

FIG. 8 is a schematic view illustrating one aspect of the method for detecting a nucleic acid according to the embodiment and illustrates a state where a first capture probe 4 and a second capture probe 4' in which the sequences of the second portion 2 are different from each other are immobilized on the substrate. The second portion of the first capture probe is referred to as a sequence A. The second portion of the second capture probe is referred to as a sequence B. A first detection probe 5 is capable of being hybridized with the sequence A and a second detection probe 15 is capable of being hybridized with the sequence B.

The miRNA capable of hybridizing with the first portion of the first capture probe 4 (hereinafter, referred to as a first miRNA) and the miRNA capable of hybridizing with the first portion of the second capture probe 4' (hereinafter, referred to as a second miRNA) are different types of miRNA. Furthermore, the amount of the first miRNA in the solution is considerably greater than the amount of the second miRNA in the solution. At this time, if the sequence A and the sequence B are the same as each other and the common detection probe is used, the amount of the detection probe that is preferable to the first miRNA may be an excessive amount of the detection probe to the second miRNA, and a possibility of hybridization of a non-specific detection probe is increased in detecting the second miRNA.

Thus, by using the first capture probe and the second capture probe in which the sequences of the second portion 2 are different from each other and the plurality of types of detection probes corresponding thereto, it is possible to select a preferable amount of the detection probe, which is adapted to the amount of the miRNA.

In addition, the first detection probe and the second detection probe may be labeled with a labeling substance that is different from each other or in a different amount. At this time, the labeling substance preferably corresponds to the sequence of the second portion.

As illustrated in FIG. 8, the first detection probe 5 is labeled with a labeling substance 5a and the second detection probe 15 is labeled with a labeling substance 5a'.

Since the first detection probe and the second detection probe are labeled by a labeling substance that is different from each other, in a case where the amount of the first detection probe and the second detection probe is changed, a signal can be distinguished for each labeling type, and accordingly the amount of the miRNA as a detection target can be determined more accurately.

In a case where the first detection probe and the second detection probe are labeled with a labeling substance that is the same as each other and in a different amount, labeling intensity can be increased and the target miRNA can be detected even more sensitively.

In addition, as exemplified above, in a case where the amount of the first miRNA in the solution is considerably greater than the amount of the second miRNA in the solution, the amount of the detection probe 15 hybridized with the capture probe 4' is smaller than the amount of the detection probe 5 hybridized with the capture probe 4. As a result, a difference between the signal amount originated from the detection probe 15 and the signal amount originated from the detection probe 5 becomes greater and it may be difficult to quantify the both signals at one time of the analysis. Thus, the intensity originated from the labeling substance of the first detection probe and the intensity originated from the labeling substance of the first detection probe may be different from each other.

For example, in a case where the amount of the first miRNA is greater than the amount of the second miRNA and the amount of the second miRNA is smaller than the amount of the first miRNA, labeling the first and second detection probes with a labeling substance that is different from each other, such that the signal intensity originated from the labeling of the first detection probe is greater than the signal intensity originated from the labeling of the second detection probe is exemplified. At this time, a difference between the signal intensity originated from the labeling of the first detection probe and the signal intensity originated from the labeling of the second detection probe is not particularly limited, and a difference between the signal intensities may be adjusted to be within a dynamic range of the detecting apparatus. As such, as the first detection probe and the second detection probe are labeled by a labeling substance that is different from each other or in a different amount, the detectable range of the miRNA can be expanded.

### <Capture probe, Detection probe set, Microarray, Nucleic acid detection kit, and Nucleic-acid-immobilized solid phase>

The capture probe of the embodiment is a capture probe characterized to have the first portion including a sequence capable of hybridizing with the nucleic acid as a detection target, the second portion including a sequence capable of hybridizing with the detection probe labeled with a labeling substance, and the stem portion for forming a double strand, and the first portion and the second portion are preferably disposed so as to be adjacent to each other and the first portion is preferably positioned between the second portion and the stem portion. The capture probe of the embodiment is represented by the aforementioned capture probe 4.

As another embodiment, the capture probe is preferably a probe in which the detection probe labeled with a labeling substance is hybridized with the second portion.

The detection probe set of the embodiment is a detection probe set including a plurality of types of detection probes, and the detection probe can be bound to the second portion of the capture probe, which has the first portion including a sequence capable of hybridizing with the nucleic acid as a detection target, the second portion including a sequence capable of hybridizing with the detection probe, and the stem portion for forming a double strand and includes the first detection probe and the second detection probe labeled with a labeling substance that is different from each other or in a different amount. The detection probe set is represented as a probe set including the first detection probe 5 and the second detection probe 15 labeled with a labeling substance that is different from each other as illustrated in FIG. 8. The labeling substance preferably corresponds to the sequence of the second portion. Due to this configuration, it is possible to distinguish each miRNA exactly.

The microarray of the embodiment is a microarray in which a plurality of capture probes is immobilized on a substrate, the capture probe including the first portion including a sequence capable of hybridizing with the nucleic acid as a detection target, the second portion including a sequence capable of hybridizing with the detection probe labeled with a labeling substance, and the stem portion for forming a double strand.

The capture probe of the embodiment may have a spacer at the end of a side bonded to the substrate and may include the first portion, the second portion, the stem portion having a loop structure, and the spacer, and as the microarray of the embodiment, the microarray described in the aforementioned <Method for detecting a nucleic acid> can be exemplified.

As another embodiment, the detection probe labeled with a labeling substance may be hybridized in advance with the second portion of the capture probe. The capture probe described in the [Second Embodiment] of the <Method for detecting a nucleic acid> can be exemplified as the above.

The nucleic acid detection kit of the embodiment is a nucleic acid detection kit including the capture probe and the detection probe, the capture probe has the first portion including a sequence capable of hybridizing with the nucleic acid as a detection target, the second portion including a sequence capable of hybridizing with the detection probe, and the stem portion for forming a double strand, and the detection probe is labeled with a labeling substance. The capture probe of the embodiment is represented as the aforementioned capture probe 4. The detection probe of the embodiment is represented as the aforementioned detection probe 5.

The nucleic-acid-immobilized solid phase of the embodiment is a nucleic-acid-immobilized solid phase in which the nucleic acid-detection probe-capture probe complex is immobilized on a solid phase, the a nucleic acid-detection probe-capture probe complex is formed by linking the detection probe and the capture probe via the nucleic acid as a detection target, the capture probe has the first portion including a sequence capable of hybridizing with the nucleic acid as a detection target, the second portion including a sequence capable of hybridizing with the detection probe, and the stem portion for forming a double strand, and the detection probe and the nucleic acid, and the nucleic acid and the capture probe are linked to each other. The nucleic-acid-immobilized solid phase of the embodiment can be preferably used in the step (f) described in the [Fifth Embodiment] of the <Method for detecting a nucleic acid>.

The nucleic-acid-immobilized solid phase obtained by subjecting the steps (a) to (d) described in the <Method for detecting a nucleic acid> is exemplified as the nucleic-acid-immobilized solid phase of the embodiment, but the nucleic-acid-immobilized solid phase is not limited to subjecting the above steps. Among these, the nucleic-acid-immobilized solid phase, in which the nucleic acid-detection probe-capture probe complex is immobilized on a solid phase and which is obtained by subjecting the steps (a) to (d) of the [First Embodiment], can be exemplified as a preferred example, the nucleic acid-detection probe-capture probe complex is formed by ligating the 5' end of the capture probe 4 and 3' end of the miRNA 6, and the 5' end of the miRNA 6 and the 3' end of the detection probe 5.

### <Fluid device>

The fluid device of the embodiment (for example, a microfluid device (micro TAS), a milli fluid device (milli TAS), or the like) is a fluid device including a nucleic acid detection portion that has a substrate in which a capture probe is immobilized, and the capture probe has the first portion including a sequence capable of hybridizing with the nucleic acid as a detection target, the second portion including a sequence capable of hybridizing with the detection probe labeled with a labeling substance, and the stem portion for forming a double strand. In addition, in the fluid device of the embodiment, the detection probe labeled with a labeling substance may be hybridized in advance with the second portion.

As the substrate formed by the capture probe being immobilized, the previously described microarray can be exemplified.

FIG. 9 is a schematic view of a configuration of a fluid device according to the embodiment. A fluid device 101 includes a nucleic acid detection portion 104 which has a substrate 104c formed by the capture probe being immobilized and an inlet for introducing a detection probe 104a. The nucleic acid detection portion 104 may further include an inlet for introducing a sample 102b for introducing a nucleic acid sample.

Hereinafter, the present invention is described using Examples, but the present invention is not limited to the following Examples.

### [Examples]

### [Example 1]

A RNA having a sequence of miR-16 was synthesized as the target miRNA. In addition, a detection probe having a T7 promoter sequence was synthesized. Then, a capture probe having a sequence complementary to the sequence of miR-16 and a sequence complementary to the T7 promoter sequence was designed and synthesized.

The used sequences of the target miRNA, the capture probe, and the detection probe are shown as follows.

### (1) Target miRNA: miR-16 [Sequence: 5'-p-X1-3']

p represents a phosphoric acid and X1 represents the following sequence. The following sequence of the target miRNA is RNA.

UAGCAGCACGUAAAUAUUGGCG (SEQ ID No. 1: 22 mer)

### (2) Detection probe 1 (Detect probe 1) [Sequence: 5'-p-Al-X2-3']

p represents a phosphoric acid, A1 represents Alexa647-AminoC6-dA, and X2 represents the following sequence. The following sequence of the detection probe 1 is RNA and 2'-O-methyl RNA. The sequence represented by capital letters is RNA and the sequence represented by small letters is 2'-O-methyl RNA.

X2: GcUaGuUaUuGcUcAgCgG (SEQ ID No. 2: 19 mer)

### (3) Capture probe 1 (Capture probe 1) [Sequence: 5'-p-X3-X4-fN-3']

p represents a phosphoric acid, X3 represents the following sequence, X4 represents the following sequence, f represents 6-FAM(6-fluoroscein), and N represents an amino group. X4 is a sequence that plays a roll of the spacer. The following sequence of the capture probe 1 is DNA. 
X3:

CTCAACTGGTAATTCAGTTGAGCGCCAATATTTACGTGCTGCTACCGCTGAGC

AATAACTAGC (SEQ ID No. 3: 63 mer) X4: ACAACAACAACAACAACAACA
 (SEQ ID No. 4: 21 mer)

A solution including the capture probe shown in Table 1 was discharged on a glass substrate (Nexterion Slide epoxy coated substrate) using an inkjet device (manufactured by MicroJet Corporation, LaboJet-500Bio). In Table 1, a composition of 20 x SSC buffer is 3M NaCl and 0.3M sodium citrate.

**[Table 1]**

| | |
|---|---|
| 20 *µ*M Capture probe 1 | 75 *µ*l |
| 5M betaine | 45 *µ*l |
| 20 × SSC buffer | 22.5 *µ*l |
| DEPC-Treated Water | 7.5 *µ*l |
| Total | 150 *µ*l |

The temperature of the obtained substrate was maintained under conditions of 25°C and high humidity (within a chamber with 20 µl of water at 50°C set therein) for one night and the capture probe-1 was reacted with an epoxy group of the glass substrate. Thereafter, the substrate was cleansed by 0.1% TritonX-100, 1 mM HCl, 100 mM KCl, ultrapure water, a blocking solution (100 mM Tris-HCl pH 9.0, 50 mM ethanolamine, 0.1 % SDS), and ultrapure water, and dried by air so as to obtain a microarray in which the capture probe-1 was immobilized on the glass substrate. The immobilized density of the capture probe calculated from the fluorescence intensity of 6-FAM on the substrate was 1,540 copy/µm².

A hybridization reaction solution 1 of a control, not including the miRNA-16 was prepared as shown in Table 2.

**[Table 2]**

| | |
|---|---|
| 3 nM miRNA-16 | 100 *µ*l |
| 20 µM Detect probe 1 | 1.5 *µ*l |
| 1M Tris-HCl (pH7.5) | 20 *µ*l |
| 1M MgCl₂ | 3 *µ*l |
| 100 mM ATP | 0.3 *µ*l |
| 50 mg/ml BSA | 3 *µ*l |
| 1M DTT | 3 *µ*l |
| 2. 5M NaCl | X *µ*l |
| DEPC-Treated Water | 169.2-X *µ*l |
| Total | 300 *µ*l |

A hybridization reaction solution 2 was prepared according to the same composition as the hybridization reaction solution 1 except that the miR-16 was contained at a concentration of 1 nM.

A hybridization reaction solution 3 was prepared according to the same composition as the hybridization reaction solution 1 except that NaCl was contained at a concentration of 150 nM.

A hybridization reaction solution 4 was prepared according to the same composition as the hybridization reaction solution 2 except that the miR-16 was contained at a concentration of 1 nM.

A hybridization reaction solution 5 was prepared according to the same composition as the hybridization reaction solution 4 except that NaCl was contained at a concentration of 75 nM.

After each hybridization reaction solution was incubated at 95°C for 5 minutes, the temperature returned to room temperature and T4 DNA ligase was added to the incubated hybridization reaction solution such that the final concentration became 5 units/µl. Subsequently, this hybridization reaction solution was sealed in a state of being in contact with the microarray substrate and hybridized at 37°C for 2 hours while shaking on a shaker at a rate of 1,000 rpm.

After the hybridization reaction was completed, the microarray substrate was cleansed while shaking in a cleansing solution (50% Formamide, 0.1 % SDS, 10 mM EDTA) for 3 minutes and this cleansing operation was repeated twice. After the microarray substrate was rinsed by ultrapure water, the substrate was dried and observed by a fluorescence microscope to measure the fluorescence intensity.

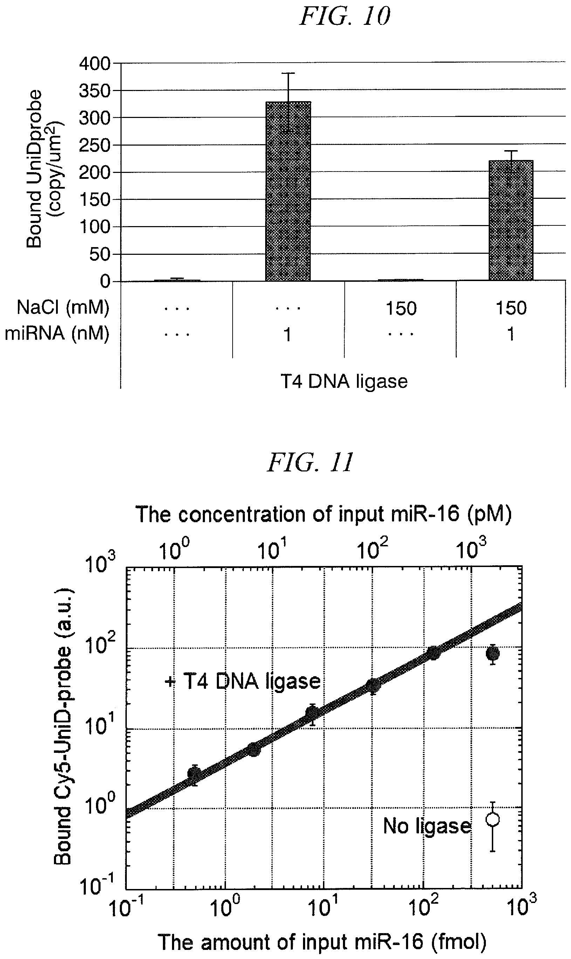

The result is shown in FIG. 10. As illustrated in graph of FIG. 10, a fluorescent image of the detection probe labeled with Alexa647 dependent on the miRNA was observed. In addition, as the concentration of NaCl was lower, a fluorescent signal of the detection probe was increased.

### [Example 2]

Next, a change in the fluorescence intensity of the detection probe in each spot was confirmed when the concentration of the miR-16 was changed. Reaction solutions 6 to 11 were prepared such that each of the reaction solution contains the miR-16 at the final concentration of 0.488 fM, 1.95 fM, 7.81 fM, 31.3 fM, 125 fM, and 500 fM. The composition of the reaction solutions 6 to 11 was the same as the reaction solution 1 except that the concentration of the miR-16 was changed.

The hybridization reaction was performed in the same manner as Example 1. At this time, a reaction in which T4 DNA ligase was not added to the reaction solution 11 (No ligase) was performed as a control reaction.

The fluorescence intensity was measured in the same manner as Example 1. The result is shown in FIG. 11. In the range of 0.488 fmol to 125 fmol of the concentration of the miR-16 used in the hybridization reaction, linearity was confirmed and it was confirmed that a signal of the fluorescence intensity of the entire spots is increased dependent on the concentration of the miRNA. When the concentration of miR-16 in the reaction solution was 125 fM and the concentration of miR-16 in the reaction solution was 500 fM, it was understood that the fluorescence intensities were the same as each other and a fluorescent signal of the detection probe bound to the capture probe was saturated. The same applied to the fluorescence intensity when the miRNA-16 was 0 nM as a control (No ligase).

From the above result, according to the embodiment, it was shown that the nucleic acid could be detected in a highly precise manner by using the detection probe and the capture probe according to the embodiment.

Each configuration in each embodiment and a combination thereof are one example and an addition, omission, substitution, and modification to others of the configuration can be performed within the range not departing from the gist of the present invention. The present invention is not limited to the embodiments and only limited by the scope of claims.

### Reference Signs List

1... first portion, 1a and 1a'...first photoreactive base derivative, 2... second portion, 3a and 3a'...stem portion, 3b...loop structure, 4 and 4'...capture probe, 4a...spacer, 5 and 15...detection probe, 5a and 5a'...labeling substance, 6...miRNA, 7...substrate, 8...T4 DNA ligase, A and B...sequence, 11...RT primer, 12...reverse transcriptase, 13...cDNA, 14 and 14'...PCR primer, 16...intercalator, 17...DNA polymerase, 20...detecting apparatus, 21...well, 23...housing, 24a...reagent inlet port, 24b...reagent outlet port, 25...spacer, 30...heater unit, 101...fluid device, 104...nucleic acid detection portion, 104a...inlet for introducing a detection probe, 104c...substrate, 102b...inlet for introducing a sample

## Claims

1. A method for detecting a nucleic acid, comprising:
(a) bringing a solution including a nucleic acid and a detection probe labeled with a labeling substance into contact with a solid phase on which a capture probe is immobilized, the capture probe having a first portion including a sequence capable of hybridizing with the nucleic acid, a second portion including a sequence capable of hybridizing with the detection probe, and a stem portion for forming a double strand;
(b) hybridizing the nucleic acid with the first portion, hybridizing the detection probe with the second portion, and forming a nucleic acid-detection probe-capture probe complex on the solid phase;
(c) ligating the nucleic acid and the detection probe in a state where the nucleic acid is hybridized with the first portion and the detection probe is hybridized with the second portion;
(d) eliminating binding between the capture probe and the detection probe that has not been ligated with the nucleic acid; and
(e) detecting the labeling substance in the nucleic acid-detection probe-capture probe complex in which the nucleic acid and the detection probe are ligated and which is formed on the solid phase.

2. A method for detecting a nucleic acid, comprising:
(a') bringing a solution including a nucleic acid into contact with a solid phase on which a capture probe is immobilized, the capture probe having a first portion including a sequence capable of hybridizing with the nucleic acid, a second portion including a sequence capable of hybridizing with a detection probe, and a stem portion for forming a double strand,
wherein the detection probe labeled with a labeling substance is hybridized with the second portion in advance;
(b') hybridizing the nucleic acid with the first portion and forming a nucleic acid-detection probe-capture probe complex on the solid phase;
(c) ligating the nucleic acid and the detection probe in a state where the nucleic acid is hybridized with the first portion and the detection probe is hybridized with the second portion;
(d) eliminating binding between the capture probe and the detection probe that has not been ligated with the nucleic acid; and
(e) detecting the labeling substance in the nucleic acid-detection probe-capture probe complex in which the nucleic acid and the detection probe are ligated and which is formed on the solid phase.

3. The method for detecting a nucleic acid according to Claim 1 or 2,
wherein the step (d) is executed by placing the capture probe under a condition capable of eliminating hybridization between the nucleic acid and the capture probe, and the detection probe and the capture probe.

4. The method for detecting a nucleic acid according to any one of Claims 1 to 3,
wherein in the step (c), the nucleic acid and the capture probe are linked to each other.

5. The method for detecting a nucleic acid according to Claim 4,
wherein the linkage of the nucleic acid and the capture probe is caused by ligation of the nucleic acid and the end of the stem portion.

6. The method for detecting a nucleic acid according to any one of Claims 1 to 5,
wherein the stem portion has a loop structure.

7. The method for detecting a nucleic acid according to any one of Claims 1 to 6,
wherein the first portion and the second portion are disposed so as to be adjacent to each other.

8. The method for detecting a nucleic acid according to any one of Claims 1 to 7,
wherein the first portion is positioned between the second portion and the stem portion.

9. The method for detecting a nucleic acid according to any one of Claims 1 to 8,
wherein a plurality of types of the capture probes is used, and the capture probe includes a first capture probe and a second capture probe in which sequences of the second portions are different from each other.

10. The method for detecting a nucleic acid according to Claim 9,
wherein a plurality of types of the detection probes is used, and the detection probe includes a first detection probe and a second detection probe which are labeled by a labeling substance that is different from each other or in a different amount.

11. The method for detecting a nucleic acid according to Claim 9 or 10, wherein the labeling substance corresponds to the sequence of the second portion.

12. The method for detecting a nucleic acid according to any one of Claims 1 to 11, further comprising:
(f) synthesizing a nucleic acid using, as a template, a nucleic acid in the nucleic acid-detection probe-capture probe complex in which the nucleic acid and the detection probe are ligated and which is formed on the solid phase.

13. The method for detecting a nucleic acid according to any one of Claims 1 to 12,
wherein the first portion and the second portion are composed of DNA, the detection probe is composed of RNA, and the nucleic acid and the end of the detection probe are ligated by T4 DNA ligase.

14. The method for detecting a nucleic acid according to any one of Claims 1 to 13,
wherein the nucleic acid is miRNA.

15. The method for detecting a nucleic acid according to any one of Claims 1 to 14,
wherein the capture probe and/or the detection probe include one or more compounds selected from the group consisting of 2'-O-methyl RNA, LNA (Locked Nucleic Acid), and BNA (Bridged Nucleic Acid).

16. A capture probe, comprising:
a first portion including a sequence capable of hybridizing with a nucleic acid as a detection target;
a second portion including a sequence capable of hybridizing with a detection probe labeled with a labeling substance; and
a stem portion for forming a double strand.

17. The capture probe according to Claim 16,
wherein the first portion and the second portion are disposed so as to be adjacent to each other, and the first portion is positioned between the second portion and the stem portion.

18. The capture probe according to Claim 16 or 17,
wherein the detection probe labeled with a labeling substance is hybridized with the second portion.

19. A detection probe set, comprising:
a plurality of types of detection probes,
wherein the detection probe can be bound to a second portion of a capture probe, and the capture probe includes a first portion including a sequence capable of hybridizing with a nucleic acid as a detection target, the second portion including a sequence capable of hybridizing with the detection probe, and a stem portion for forming a double strand, and
wherein the detection probe includes a type of a detection probe which includes a first detection probe and a second detection probe which is labeled by a labeling substance that is different from each other or in a different amount.

20. The detection probe set according to Claim 19,
wherein the labeling substance corresponds to the sequence of the second portion.

21. A microarray formed by a plurality of capture probes being immobilized on a substrate, the capture probe having a first portion including a sequence capable of hybridizing with a nucleic acid as a detection target, a second portion including a sequence capable of hybridizing with a detection probe labeled with a labeling substance, and a stem portion for forming a double strand.

22. The microarray according to Claim 21,
wherein the detection probe labeled with a labeling substance is hybridized in advance with the second portion.

23. The microarray according to Claim 21 or 22,
wherein the stem portion has a loop structure.

24. The microarray according to any one of Claims 21 to 23,
wherein the capture probe has a spacer at the end on the side binding to a substrate, and
wherein the capture probe includes the first portion, the second portion, the stem portion having a loop structure, and the spacer.

25. A nucleic acid detection kit, comprising:
a capture probe; and
a detection probe,
wherein the capture probe includes a first portion including a sequence capable of hybridizing with a nucleic acid as a detection target, a second portion including a sequence capable of hybridizing with the detection probe, and a stem portion for forming a double strand, and
wherein the detection probe is labeled with a labeling substance.

26. A nucleic-acid-immobilized solid phase in which a nucleic acid-detection probe-capture probe complex is immobilized on a solid phase, the nucleic acid-detection probe-capture probe complex is formed by linking a detection probe and a capture probe via a nucleic acid, the capture probe includes a first portion including a sequence capable of hybridizing with the nucleic acid as a detection target, a second portion including a sequence capable of hybridizing with the detection probe, and a stem portion for forming a double strand, and the detection probe and the nucleic acid, and the nucleic acid and the capture probe are linked to each other.

27. A fluid device, comprising:
a nucleic acid detection portion having a solid phase formed by a capture probe being immobilized,
wherein the capture probe includes a first portion including a sequence capable of hybridizing with a nucleic acid as a detection target, a second portion including a sequence capable of hybridizing with a detection probe labeled with a labeling substance, and a stem portion for forming a double strand.

28. The fluid device according to Claim 27,
wherein the detection probe labeled with a labeling substance is hybridized in advance with the second portion.


SEQUENCE LISTING

<110>  The University of Tokyo
       NIKON CORPORATION

<120>  Method for the detection of nucleic acid

<130>  PC-20379

<150>  JP 2014-147726
<151>  2014-07-18

<160>  4

<170>  PatentIn version 3.5

<210>  1
<211>  22
<212>  RNA
<213>  Homo sapiens

<400>  1
uagcagcacg uaaauauugg cg                                                22


<210>  2
<211>  19
<212>  RNA
<213>  Artificial Sequence

<220>
<223>  Description of Artificial Sequence: Detect probe 1


<220>
<221>  modified_base
<222>  (2)..(2)
<223>  cm

<220>
<221>  modified_base
<222>  (4)..(4)
<223>  a stands for 2'-O- methyladenosine

<220>
<221>  modified_base
<222>  (6)..(6)
<223>  um

<220>
<221>  modified_base
<222>  (8)..(8)
<223>  a stands for 2'-O- methyladenosine

<220>
<221>  modified_base
<222>  (10)..(10)
<223>  um

<220>
<221>  modified_base
<222>  (12)..(12)
<223>  cm

<220>
<221>  modified_base
<222>  (14)..(14)
<223>  cm

<220>
<221>  modified_base
<222>  (16)..(16)
<223>  gm

<220>
<221>  modified_base
<222>  (18)..(18)
<223>  gm

<400>  2
gcuaguuauu gcucagcgg                                                    19


<210>  3
<211>  63
<212>  DNA
<213>  Artificial Sequence

<220>
<223>  Description of Artificial Sequence: Capture probe1-1.

<400>  3
ctcaactggt aattcagttg agcgccaata tttacgtgct gctaccgctg agcaataact      60

agc                                                                     63


<210>  4
<211>  21
<212>  DNA
<213>  Artificial Sequence

<220>
<223>  Description of Artificial Sequence: Capture probe1-2.

<400>  4
acaacaacaa caacaacaac a                                                 21